# EUROPÄISCHE PATENTSCHRIFT

(11) **EP 1 207 960 B1**
(45) Veröffentlichungstag und Bekanntmachung des Hinweises auf die Patenterteilung: **07.01.2004**
(21) Anmeldenummer: 00949377.6
(22) Anmeldetag: 19.07.2000
(51) Int. Cl.: B01L 3/02, B01J 19/00

(54) **NADEL UND VERFAHREN ZUM TRANSFER VON LIQUIDEN SOWIE VERFAHREN ZUM HERSTELLEN DER NADEL**
NEEDLE, METHOD FOR TRANSFERRING LIQUIDS AND METHOD FOR PRODUCING SAID NEEDLE
AIGUILLE ET PROCEDE POUR LE TRANSFERT DE LIQUIDES AINSI QUE PROCEDE DE FABRICATION DE CETTE AIGUILLE

(30) Priorität: 20.07.1999 DE 19933838
(43) Veröffentlichungstag der Anmeldung: 29.05.2002
(73) Patentinhaber: Deutsches Ressourcenzentrum für Genomforschung GmbH, 14059 Berlin (DE)
(72) Erfinder: BIENERT, Klaus, D-14450 Gross Kreutz (DE); KRAACK, Heiko, D-14469 Potsdam-Bornstedt (DE); VENTE, Andreas, D-10707 Berlin (DE); ZETTL, Rolf, D-10439 Berlin (DE); PFLEGING, Wilhelm, D-76344 Eggenstein-Leopoldshafen (DE); BESSER, Heino, D-76344 Eggenstein-Leopoldshafen (DE); RADELOF, Uwe, D-14469 Potsdam (DE)
(74) Vertreter: VOSSIUS & PARTNER
(86) Internationale Anmeldenummer: PCT/EP2000/006912
(87) Internationale Veröffentlichungsnummer: WO 2001/005506

(56) Entgegenhaltungen:
- WO-A-97/14967
- US-A- 3 252 331
- US-A- 3 650 306
- US-A- 5 158 101
- US-A- 5 770 151

## Beschreibung

Die vorliegende Erfindung betrifft eine Nadel und ein Verfahren zum Transfer von Liquiden sowie ein Verfahren zum Herstellen der Nadeln.

Insbesondere betrifft die Erfindung eine Nadel und ein Verfahren zum Transfer von in einem Muster angeordnetem biologischen Material, wobei man das biologische Material mit an einem Roboterkopf angebrachten Nadeln in Kontakt bringt und das biologische Material auf einen Träger überträgt. Sofern das biologische Material in einem Muster angeordnet ist, sind die an dem Roboterkopf angebrachten Nadeln nach dem selben Muster angeordnet. Es ist ferner bevorzugt, daß das Muster dem Muster der Anordnung der Vertiefungen in einer Mikrotiterplatte entspricht. Insbesondere ist der Roboterkopf ein Bestandteil eines Pickingroboters und/oder eines Spottingroboters. Die Erfindung betrifft femer ein Verfahren zur Herstellung der erfindungsgemäßen Nadeln und die Verwendung der Nadeln zum Transfer von dem beispielsweise in einem Muster angeordneten biologischen Material auf einen Träger.

Computerassistierte Screening-Verfahren finden immer weiteren Eingang in biologisch oder biochemisch ausgerichtete Laboratorien. Wie exemplarisch am Humangenomprojekt dargestellt, besteht ein Bedarf an Verfahren und Gerätschaften zum Nachweis und Katalogisieren von mehr Material in kürzeren Zeitabschnitten. Zum Screenen von Genbanken wurden in den letzten Jahren Roboter entwickelt, mit denen ein systematisches Absuchen der Banken und eine nachfolgende systematische Auswertung erheblich erleichtert wurde. Die in diesem Verfahren eingesetzten Roboter werden allgemein als Picking-/Spotting-Roboter bezeichnet. Die gegenwärtig eingesetzten Picking-/Spotting-Roboter sind in der Lage, biologisches Material aufzunehmen und gezielt zu überführen beziehungsweise zu verteilen. Hierbei werden Gadgets (Nadelmatrizen) in verschiedenen Ausführungen verwendet, beispielsweise im Rasterformat 8 x 12 oder 16 x 24. Diese Nadelmatrizen sind mit Nadeln, vorzugsweise Edelstahlnadeln ausgerüstet. Die Edelstahlnadeln haben eine gute Korrosionsbeständigkeit.

Die gegenwärtig eingesetzten Spottingroboter sind in der Lage, Flüssigkeiten, wie zum Beispiel PCR-Produkte oder kultivierte Zellen und Mikroorganismen, aus einer Mikrotiterplatte (üblicherweise im Raster 8 x 12 (96 Vertiefungen) oder 16 x 24 (384 Vertiefungen)) aufzunehmen und gezielt auf Trägermaterialen abzulegen. Hierbei werden die vorstehend bereits erwähnten Gadgets (Nadelmatrizen mit Raster entsprechend der Mikrotiterplatte) verwendet, wobei in einem Arbeitsschritt (Aufnehmen der Klone und Aufbringen auf dem Träger) 96 beziehungsweise 384 Klone auf den Träger transferiert werden können.

Die Spitzen der im Gadget angeordneten Nadeln haben üblicherweise einen Durchmesser von 100 bis 400 µm. Durch Eintauchen dieser Nadeln in einen Reaktionsbehälter, wie zum Beispiel eine Mikrotiterplatte, kann in Form von Spots in einem Arbeitsschritt genügend biologisches Material von dem Reaktionsbehälter auf die Trägermatrix übertragen werden. Um mehrere, identische Spots auf die Trägermaterialien ausbringen zu können, ist jeweils eine zeitaufwendige Verfahrbewegung des Roboterkopfes zum Aufnehmen von Material aus der Mikrotiterplatte notwendig.

Das Ziel. aller Hochdurchsatzsysteme ist jedoch ein möglichst hoher Systemdurchsatz. Bei Robotersystemen können durch möglichst kurze Verfahrwege höhere Durchsätze erzielt werden. Aus diesem Grund wurden Nadeln entwickelt, die in der Lage sind, mehr Material aus einer Mikrotiterplatte in einem Schritt aufzunehmen und dieses mehrfach auf die Trägermaterialien zu überführen.

Eine derartige Nadel wird beispielsweise in der US-A-5,807,522 unter Bezugnahme auf ein Verfahren und eine Vorrichtung zum Ausbilden eines Musters von biologischen Proben auf einem Träger offenbart. Die dort beschriebene Nadel hat einen länglichen, nach unten offenen Kapillarkanal zur Aufnahme einer bestimmten Menge Reagenzlösung. Der Kapillarkanal ist durch ein paar voneinander beabstandete Elemente gebildet, deren Abstand sich zur Spitze der Nadel hin verringert. In anderen Worten ist bei dieser Art von Nadeln zumindest die Spitze der Nadel durch einen durchgehenden Schlitz in zwei Hälften mit einem dazwischen liegenden Zwischenraum geteilt, der unter Kapillarwirkung die zu transferierende Flüssigkeit aufnimmt.

Derartige Nadeln sind beispielsweise auch im Art. "RNA-Expressionsanalyse auf cDNA-Arrays", Holger Eickhoff et al. in Medgen 11 (1999) beschrieben. In dem Artikel wird beschrieben, daß die einzelnen Nadeln (Pins) des Stempels stumpf oder geschlitzt sein können. Die PCR-Flüssigkeit in den Mikrotiterplatten wird dabei entweder durch Adhäsion an den Nadeln gehalten oder durch Kapillarwirkung in den Nadelkopf hineingesogen. Durch einen Kontaktschluß der Nadelspitze mit der Oberfläche wird abhängig von der Geometrie der Nadel eine Flüssigkeitsmenge zwischen 2 und 10 nl übertragen.

Eine andere Art von Nadeln wird im Art. "Herstellen von biomolekularen Arrays - eine technologische Herausforderung" von Eugen Ermantraut in Medgen 11 (1999) beschrieben. Die dort beschriebenen Nadeln verfügen meistens über eine Kerbe, die, vergleichbar der Feder in einem Füllfederhalter, als Depot für die Lösung wirkt.

In den US-A-4 162 896, JP-A-08-23958 (Abstract), DE-C-36 30 866, US-A-5 770 151, US-A-4 687 746, US-A-5 262 128, US-A-3 252 331 und US-A-3 077 780 sind weitere Vorrichtungen zum Transfer von Flüssigkeiten beschrieben, die jedoch keine zufriedenstellenden Ergebnisse liefern.

Der vorliegenden Erfindung liegt die Aufgabe zugrunde, die aus dem bekannten Stand der Technik bekannten Nadeln und Verfahren zum Transfer von Flüssigkeiten sowie das Herstellverfahren für die Nadeln zu verbessern. Diese Aufgabe wird mit den Merkmalen der Patentansprüche gelöst.

Dabei geht die Erfindung von dem Grundgedanken aus, in mindestens einem Abschnitt oder Bereich eines Körpers oder einer Nadel, wie z.B. im Bereich der Nadelspitze und/oder zumindest teilweise im daran anschließenden Nadelschaft, mindestens eine Oberflächen kapillare zur Aufnahme des zu transferierenden Liquids vorzusehen. Vorzugsweise sind an der Nadel mehrere sich im wesentlichen in Längsrichtung der Nadel erstreckende Oberflächenkapillaren um deren Umfang angeordnet. Die erfindungsgemäße Nadel besteht beispielsweise vollständig aus Metall, Kunststoff, Keramik, Glas oder Mischungen daraus. Darüber hinaus kann die Nadel aus mehreren Schichten verschiedener Materialien aufgebaut sein. Beispielsweise kann die erfindungsgemäße Nadel einen Metallkern aufweisen, der mit einer Kunststoffbeschichtung ummantelt ist, in der die Oberflächenkapillare ausgebildet ist. Die Querschnittsform des Körpers bzw. der Nadel kann beliebig sein, ist jedoch bevorzugt rund.

Die erfindungsgemäße Nadel unterscheidet sich von den bislang in Hochdurchsatz-Systemen benutzten Nadeln insbesondere durch die aufgebrachte Oberflächenkapillare. Ein mit den erfindungsgemäßen Nadeln ausgerüstetes Gadget ist in der Lage beliebige, in gelöster Form vorliegende Substanzen, Zellen oder Mikroorganismen aus einem Reaktionsbehälter, typischerweise einer Mikrotiterplatte in einem Arbeitsschritt aufzunehmen und dann in einem einzigen Verfahrschritt mehrfach auf Trägermatrizen auszubringen. So können beispielsweise in einer Mikrotiterplatte mit 384 Vertiefungen syntetisierte Nukleinsäuren durch eine Nadelmatrix entnommen werden und in einer möglichst dichten Anordnung mehrfach auf Nylonträgermembranen, Glasträgern oder beliebigen anderen Trägern ausgebracht werden.

Gegenüber den bekannten Nadeln bietet die erfindungsgemäße Nadel insbesondere den Vorteil, daß im Vergleich zu unbehandelten Nadeln durch die Oberflächenkapillare mehr flüssiges Material aufgenommen und dementsprechend auch mehr Material an eine Trägermatrix abgegeben werden kann. Das mehrfache Ausbringen entweder auf unterschiedliche Trägermatrizen oder auf den selben Spot (Punkt des durch das Gadget erzeugten Mikroarrays) einer einzigen Trägermatrix ist ein elementarer Verfahrensschritt bei der Erstellung von Matrizen für das Hochdurchsatz-Screening. So wird beispielsweise die Anzahl der möglichen Hybridisierungen von Hochdichtefiltern mit PCR-Produkten mit der bis zu zehnfachen Aufbringung der PCR-Produkte auf den gleichen Spot erhöht. Gleichzeitig wird hierdurch eine sehr viel gleichmäßigere Mengenverteilung der Materialien in den verschiedenen Spots erzielt. Durch die erfindungsgemäße Nadel mit Oberflächenkapillare wird die Produktion solcher Filter erheblich beschleunigt, da in weniger Verfahrensschritten des Roboterarms wesentlich gleichmäßiger mehr Material auf die Matrix transferiert werden kann. Dies ist insbesondere auf eine Kombination der durch Adhäsion und Kapillarwirkung an der Nadel gespeicherten, erhöhten Flüssigkeitsmenge zurückzuführen. Da beim gesamten Produktionsprozess die Verfahrschritte des Roboterarms den größten Zeitanteil beanspruchen, kann durch eine Vergrößerung der durch die Nadel transferierbaren Flüssigkeitsmenge die Prozessdauer erheblich reduziert werden.

Die erfindungsgemäße Nadel mit Oberflächenkapillare kann beispielsweise durch Behandlung in einem Uitraschallbad, das in die zur Zeit benutzten Robotersysteme leicht integrierbar ist, einfach und schnell-gereinigt-und somit für den nächsten Transferprozess vorbereitet werden. Dieses wenig aufwendige und kostengünstige Verfahren bietet klare Vorteile gegenüber anderen zur Zeit benutzten Kombinationen von Nadel- und Reinigungssystemen, die beispielsweise mit Vakuumtechnik betrieben werden müssen.

Darüber hinaus ist ein wichtiger Vorteil der erfindungsgemäßen Nadeln, daß sie gegenüber bekannten Nadeln wesentlich kostengünstiger herstellbar sind. Bei den aus dem Stand der Technik bekannten Nadeln wird durch Elektro-Erodieren ein durchgehender Schlitz in die Nadeln eingebracht, was ein teurer und zeitaufwendiger Herstellungsprozess ist. Im Gegensatz dazu können die erfindungsgemäßen Nadeln beispielsweise durch Laseroberflächen-Strukturierung mit einer oder mehreren Oberflächenkapillaren versehen werden, was einen wesentlich kostengünstigeren Herstellungsprozess darstellt. Insbesondere bei der Verwendung einer mit einer Kunststoffschicht überzogenen Metallnadel ist diese Art der Fertigung äußerst effizient, da die Kunststoffschicht sich hervorragend mit Lasern bearbeiten läßt. Darüber hinaus kann bei dieser Ausführungsform der Nadelgrundkörper wieder verwendet werden. Die erfindungsgemäße Nadel kann somit aus jeder herkömmlichen Nadel (Spotting-Pin) einschließlich der geschlitzten Ausführungsform hergestellt werden.

Die erfindungsgemäßen Metallnadeln mit Oberflächenkapillaren werden vorzugsweise in den folgenden Bereichen eingesetzt: Die Ausbringung von kultivierten Zellen oder Mikroorganismen auf Nylonfiltern oder anderen Trägermatrizen. Diese werden im herkömmlichen System zweifach pro Filter (Duplikate) aufgebracht, um Nachweisverfahren sicherer zu gestalten. Außerdem stellt das Ausbringen von gelösten oder flüssigen Substanzen, wie beispielsweise gereinigte Nukleinsäuren, PCR-Produkte, Proteine auf Trägermatritzen wie PVDF-Membranen, Nylonmembranen, behandelte oder unbehandelte Glasoberflächen sowie andere geeignete Trägermaterialen eine andere bevorzugte Anwendung dar. Durch das im Vergleich zu herkömmlichen Nadeln signifikant höhere Volumen der transferierten Lösung bietet sich ebenfalls eine Anwendung in der Synthese von Molekülen auf Trägermaterialen an. So können die erfindungsgemäßen Nadeln beispielsweise zur Synthese von Peptiden oder Oligonukleotiden auf Filtern oder anderen geeigneten Matrizen Anwendung finden. Der Einsatz von Nadeln mit Oberflächenkapillaren und das dadurch mögliche mehrfache Übertragen der gewünschten Substanzen und biologischen Materialien in einem Verfahrensschritt erhöht in jeder dieser beschriebenen Anwendungen den Systemdurchsatz und/oder die Qualität der erstellten Materialien wesentlich.

Das biologische Material umfaßt insbesondere Nukleinsäure, (Poly)peptide oder transformierte Wirtsorganismen, wobei die transformierten Wirtsorganismen Hefen, vorzugsweise Pistoria oder Saccharomyces-Zellen, Bakterien, vorzugsweise E. coli, Insektenzellen, vorzugsweise Spodoptera frugiperda-Zellen, Pilzzellen, vorzugsweise Aspergilus-Zellen, Pflanzenzellen oder Säugerzellen sind.

Eine andere bevorzugte Anwendung der erfindungsgemäßen Nadeln ist die Vervielfältigung von Mikrotiterplatten mit schwierig kultivierbaren Zellen oder Mikroorganismen. Bei einem Transfer dieser Organismen mit den erfindungsgemäßen Nadeln wird mehr biologisches Material in die Tochterplatten übertragen. Dadurch ist eine insgesamt wesentlich reproduzierbarere Vervielfältigung gewährleistet.

Die erfindungsgemäße Nadel wird im folgenden anhand bevorzugter Ausführungsformen unter Bezugnahme auf die Zeichnungen beispielhaft beschrieben. Es zeigen:
- Figur 1: eine schematische Ansicht der erfindungsgemäßen Nadel;
- Figur 2: eine vergrößerte Darstellung einer ersten Ausführungsform der erfindungsgemäßen Nadel aus einem einheitlichen Material;
- Figur 3: eine vergrößerte Darstellung einer zweiten Ausführungsform der erfindungsgemäßen Nadel mit einem Nadelgrundkörper und einem Überzugmaterial;
- Figur 4: eine Ansicht-auf die-Spitze der erfindungsgemäßen Nadel; und
- Figur 5: eine ausschnittsweise Vergrößerung der Ansicht auf die Spitze der Nadel von Figur 4.

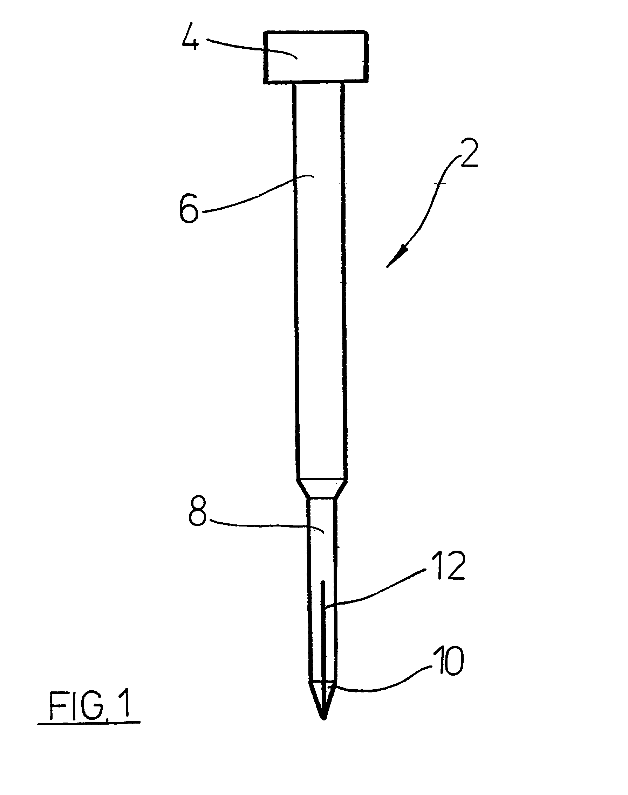

Die in Figur 1 schematisch dargestellte erfindungsgemäße Nadel 2 weist einen Nadelkopf 4 an einem Ende der Nadel auf, das beispielsweise in einem (nicht dargestellten) Spottingroboter beziehungsweise ein Gadget einsetzbar ist. Der Nadelkopf 4 ist dabei vorzugsweise derart ausgebildet, daß er je nach Verwendungszweck an einer Haltevorrichtung montierbar ist. Ein derartiger Nadelkopf 4 ist jedoch nicht zwingend erforderlich, da die erfindungsgemäße Nadel 2 beispielsweise auch am Nadelschaft 6 gehalten werden kann. In der in Figur 1 dargestellten Ausführugsform der erfindungsgemäßen Nadel 2 schließt sich der Nadelschaft 6 an den Nadelkopf 4 an. Der Nadelschaft 6 ist vorzugsweise in einem dem Nadelkopf 4 gegenüberliegenden Endabschnitt 8 verjüngt oder abgesetzt ausgebildet. An dem dem Nadelkopf 4 gegenüberliegenden Ende des abgesetzten Schaftabschnitts 8 ist eine Nadelspitze 10 ausgebildet.

Vorzugsweise ist die erfindungsgemäße Nadel 2 integral aus einem gemeinsamen Grundkörper ausgebildet. Der in der Figur 1 dargestellte Nadelkopf 4 weist beispielsweise eine Länge von 2,0 mm und einen Durchmesser von 4,0 mm auf. Der Nadelschaft 6 hat in dieser Ausführungsform eine Länge von 16,0 mm und einen Durchmesser von 2,0 mm. Der verjüngte beziehungsweise abgesetzte Schaftabschnitt 8 weist eine Länge von 8,0 mm und einer Durchmesser von 1,0 mm auf. Die daran anschließende Nadelspitze 10 ist 2,0 mm lang und hat einen Spitzendurchmesser im Bereich zwischen 100 µm und 400 µm. Diese Abmessungen beschreiben lediglich eine beispielhafte Dimensionierung der erfindungsgemäßen Nadel. Andere Abmessungen sind selbstverständlich möglich.

Die erfindungsgemäße Nadel 2 weist ferner mindestens eine Oberflächenkapillare 12 auf. Die Oberflächenkapiltare 12 erstreckt sich, wie in Figur 1 dargestellt, vorzugsweise in Längsrichtung der Nadel 2 zumindest im Bereich der Nadelspitze 10, bevorzugt jedoch auch am abgesetzten Schaftabschnitt 8, kann jedoch auch schräg zur Längsrichtung verlaufen und/oder Abzweigungen beziehungsweise eine Verästelung aufweisen. Darüber hinaus werden bevorzugt mehrere Oberflächenkapillaren 12 im bzw. am Umfang der erfindungsgemäßen Nadel 2 angeordnet bzw. ausgebildet. Die axiale Länge der Oberflächenkapillare 4 wird insbesondere durch die Füllhöhe des Reaktionsgefäßes (Mikrotiterplatte) definiert. Die Breite der Oberflächenkapillare liegt vorzugsweise im Bereich zwischen 30 und 50 µm.

Obwohl in Figur 1 eine Nadel 2 mit abgesetztem Nadelschaft dargestellt ist, kann die Oberflächenkapillare 12 selbstverständlich auch an einer nicht abgesetzten Nadel mit im wesentlichen konstantem Schaftdurchmesser bis zur Spitze realisiert werden.

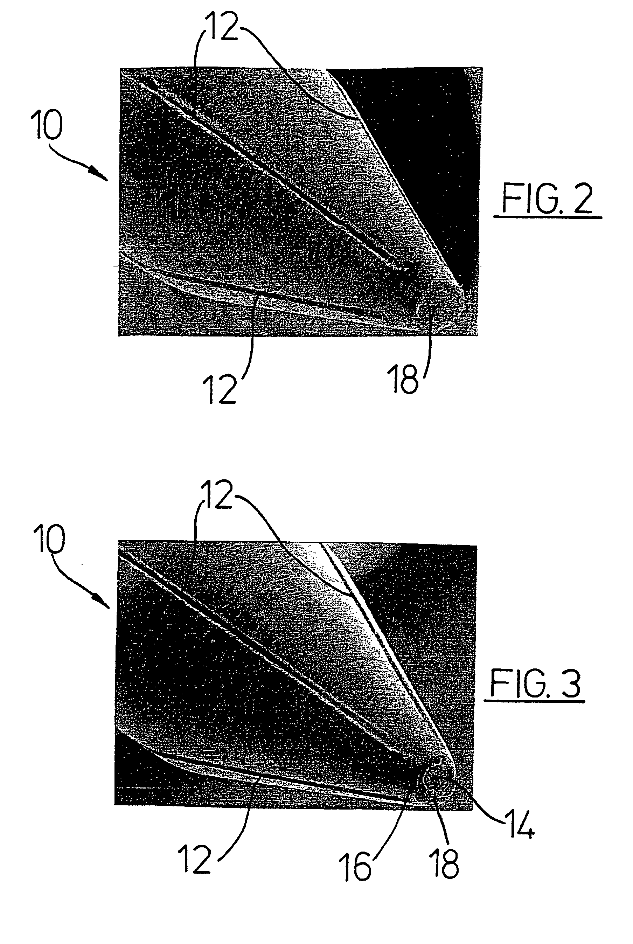

Die in den Figuren 2 und 3 gezeigte Vergrößerung der Nadelspitze 10 der erfindungsgemäßen Nadel 2 zeigt zwei unterschiedliche Ausführungsformen der Erfindung. In Figur 2 sind drei von insgesamt vier um jeweils 90 Grad versetzte Oberflächenkapillaren 12, die im wesentlichen in Längsrichtung entlang der Nadel 2 angeordnet sind dargestellt. Bei dieser Ausführungsform ist zumindest der Teil des Nadelkörpers, in dem die Oberflächenkapillaren 12 vorgesehen sind, aus einem integralen Material hergestellt. Vorzugsweise besteht die gesamte Nadel 2 dieser Ausführungsform aus einem einheitlichen Material.

Die in Figur 3 dargestellte Ausführungsform der erfindungsgemäßen Nadel 2 unterscheidet sich von der in Figur 2 gezeigten dadurch, daß auf einem Nadelgrundkörper 14 ein Überzugsmaterial 16 vorgesehen ist. Vorzugsweise ist der Nadelgrundkörper 14 aus Metall gebildet, während der Überzug 16 aus Kunststoff, wie zum Beispiel Polyamid ausgebildet ist. Der Überzug 16 kann sich im wesentlichen über die gesamte Länge der Nadel erstrecken oder aber auch nur im Bereich, in dem die Oberflächenkapillaren 12 ausgebildet sind, vorgesehen sein. Der bei dieser Ausführungsform der erfindungsgemäßen Nadel 2 vorgesehene Überzug 16 ist insbesondere aus herstellungstechnischen Gesichtspunkten vorteilhaft, da in der relativ weichen Überzugsschicht die Kerben beziehungsweise Nuten der Oberflächenkapillaren 12 auf einfache Weise beispielsweise mit einem Laser ausgebildet werden können.

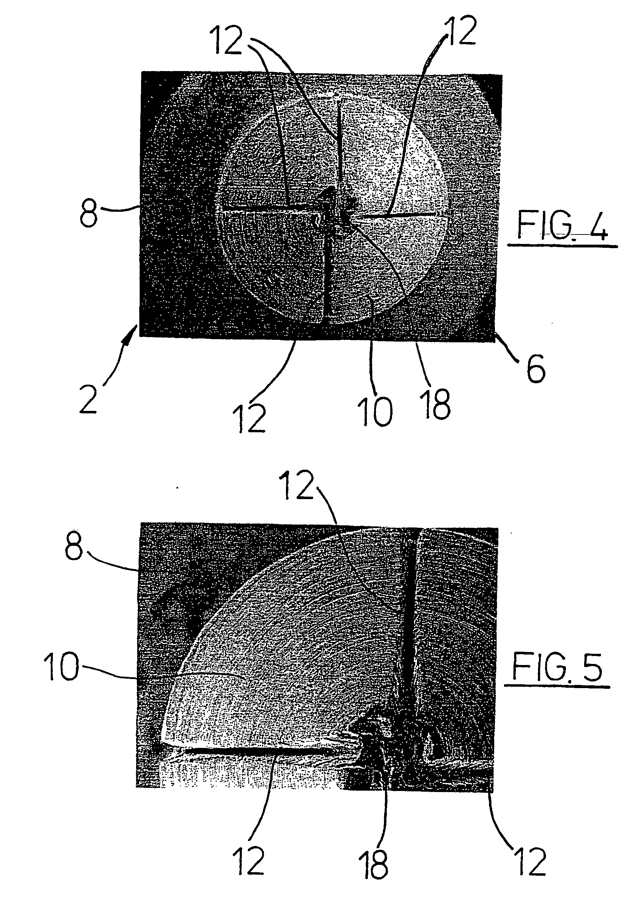

Wie in den Figuren 2 und 3 dargestellt können die Oberflächenkapillaren 12 bereits etwas vor dem Ende 18 der Spitze 10 enden oder sich aber, wie in den Figuren 4 und 5 dargestellt bis zum Ende 18 der Spitze 10 erstrecken.

Die erfindungsgemäße Nadel 2 findet insbesondere in einem Verfahren zum Transfer von biologischem Material Anwendung, wobei das biologische Material mit der an einem Roboterkopf angebrachten Nadel in Kontakt gebracht und auf einen Träger transferiert wird. Dabei speichert die erfindungsgemäße Nadel 2 die zu transportierende Flüssigkeit einerseits durch Adhäsion und andererseits durch Kapillarwirkung in den Oberflächenkapillaren 12. Dadurch lassen sich erhöhte Flüssigkeitsmengen an einer Nadel transportieren, so daß dadurch die Prozeßdauer reduziert werden kann und ein derartiges Verfahren wesentlich kostengünstiger durchgeführt werden kann.

## Patentansprüche

1. Nadel zum Transfer von Liquiden, insbesondere biologischem Material, mit mindestens einer Oberflächenkapillare (12).

2. Nadel nach Anspruch 1, die mit einem Schaft (6) und einer Spitze (10) ausgebildet ist.

3. Nadel nach Anspruch 2 mit einem abgesetzten Schaftabschnitt (8) an einem der Spitze (10) zugewandten Endabschnitt der Nadel (2).

4. Nadel nach einem der Ansprüche 1 bis 3, wobei sich die Oberflächenkapillare (12) im wesentlichen in Längsrichtung der Nadel erstreckt.

5. Nadel nach Anspruch 3 oder 4, wobei die Oberflächenkapillaren (12) im Bereich der Spitze (10) und dem abgesetzten Schaftabschnitt (8) ausgebildet ist.

6. Nadel nach einem der Ansprüche 1 bis 5, wobei mehrere Oberflächenkapillaren (12) am Umfang der Nadel angeordnet sind.

7. Nadel nach einem der Ansprüche 1 bis 6, wobei sich die Oberflächenkapillare (12) beabstandet vom freien Ende (18) der Nadel erstreckt.

8. Nadel nach einem der Ansprüche 1 bis 7, wobei die Oberflächenkapillare (12) eine Breite von 30 µm bis 50 µm aufweist.

9. Nadel nach einem der Ansprüche 1 bis 8, die integral aus einem einheitlichen Material ausgebildet ist.

10. Nadel nach einem der Ansprüche 1 bis 8, die einen Grundkörper (14) und einen zumindest im Bereich der Oberflächenkapillare (12) vorgesehenen Überzug (16) aufweist.

11. Nadel nach Anspruch 10, wobei der Grundkörper (14) aus Metall und der Überzug (16) aus Kunststoff ausgebildet ist.

12. Nadel nach einem der Ansprüche 1 bis 11, wobei die Oberflächenkapillare (12) entlang ihrer Länge im wesentlichen konstante Querschnittsdimensionen aufweist.

13. Nadel nach einem der Ansprüche 2 bis 12 mit einem an dem der Spitze (10) gegenüberliegenden Endabschnitt vorgesehenen Haltebereich (4) zur Aufnahme in einem Halteelement.

14. Nadel nach Anspruch 13, wobei der Haltebereich (4) als Nadelkopf mit einem im Vergleich zum Nadelschaft (6) größeren Durchmesser ausgebildet ist.

15. Nadelmatrix mit mindestens einer Nadel (2) nach einem der Ansprüche 1 bis 14.

16. Roboter, insbesondere zum Transfer von biologischem Material, mit mindestens einer Nadel (2) nach einem der Ansprüche 1 bis 14 bzw. einer Nadelmatrix nach Anspruch 15.

17. Roboter nach Anspruch 16, der als Picking- und/oder Spottingroboter ausgebildet ist.

18. Verfahren zum Transfer von biologischem Material mit den Schritten:
a) Bereitstellen des biologischen Materials:
b) Bereitstellen mindestens einer Nadel (2) nach einem der Ansprüche 1 bis 14, bzw. mindestens einer Nadelmaxtrix nach Anspruch 15, die mindestens eine Oberflächenkapillare (12) aufweist;
c) Inkontaktbringen der Nadel bzw, Nadelmatrix mit dem biologischen Material; und
d) Transferieren des biologischen Materials.

19. Verfahren nach Anspruch 18, bei dem die Nadel (2) an einem Roboter vorgesehen ist.

20. Verfahren nach Anspruch 19, wobei der Roboter ein Pickingroboter und/oder Spottingroboter ist.

21. Verfahren nach einem der Ansprüche 18 bis 20, wobei das biologische Material in einem Muster angeordnet wird und mit mehreren nach demselben Muster angeordneten Nadeln (2) in Kontakt gebracht wird.

22. Verfahren nach Anspruch 21, wobei das Muster der Anordnung von Vertiefungen einer Mikrotiterplatte oder dem Muster von in entsprechend regelmäßiger Anordnung gestalteten Reaktionsgefäßen entspricht.

23. Verfahren nach einem der Ansprüche 18 bis 22, wobei das biologische Material Nukleinsäure, (Poly)peptide oder transformierte Wirtsorganismen umfaßt.

24. Verfahren nach Anspruch 23, wobei die transformierten Wirtsorganismen Hefen, vorzugsweise Pistoria oder Saccharomyces-Zellen, Bakterien, vorzugsweise E. coli, Insektenzellen, vorzugsweise Spodoptera frugiperda-Zellen, Pilzzellen, vorzugsweise Aspergilus-Zellen, Pflanzenzellen oder Säugerzellen sind.

25. Verfahren nach einem der Ansprüche 18 bis 24, wobei das biologische Material auf einem flüssigen oder festen Träger angeordnet wird.

26. Verfahren nach Anspruch 25, wobei der flüssige Träger ein Kulturmedium, ein Medium für die Lagerung von biologischem Material, ein Reaktionspuffer oder eine Färbelösung ist.

27. Verfahren nach Anspruch 25, wobei der feste Träger eine Nitrocellulosemembran, eine Polyvinylidenfluoridmembran oder ein Glasträger ist.

28. Verfahren zur Herstellung einer Nadel insbesondere nach einem der Ansprüche 1 bis 14, insbesondere für ein Verfahren nach einem der Ansprüche 18 bis 27 mit den Schritten:
a) Bereitstellen eines nacle firminey Korpers; und
b) Vorsehen mindestens einer Oberflächenkapillare (12) am Körper.

29. Verfahren nach Anspruch 28, wobei der Körper eine Nadel mit einem Schaft (6) und einer Spitze (10) ist und die Oberflächenkapillare (12) zumindest im Bereich des Schaftes (6) vorgesehen wird.

30. Verfahren nach Anspruch 28 oder 29, wobei der in Schritt a) genannte Körper einen Grundkörper (14) bildet und zumindest im Bereich der Oberflächenkapillare (12) mit einem Überzug (16) versehen wird, in dem die Oberflächenkapillare (12) ausgebildet wird.

31. Verfahren nach Anspruch 30, wobei der Grundkörper (14) aus Metall und der Überzug (16) aus Kunststoff gebildet wird.

32. Verfahren nach einem der Ansprüche 28 bis 31, wobei die Oberflächenkapillare (12) durch Laserbearbeitung hergestellt wird.

33. Verfahren nach einem der Ansprüche 28 bis 32, wobei die Oberflächenkapillare (12) im wesentlichen in Längsrichtung des Körpers vorgesehen wird.

34. Verfahren nach einem der Ansprüche 28 bis 33, wobei die Oberflächenkapillare (12) beabstandet vom freien Ende (18) des Körpers vorgesehen wird.

## Claims

1. A needle for transferring liquids, especially biological material, having at least one surface capillary (12).

2. The needle according to claim 1, having a shaft (6) and a tip (10).

3. The needle according to claim 2 with a stepped shaft section (8) at an end section of the needle (2) facing the tip (10).

4. The needle according to any one of claims 1 to 3, wherein the surface capillary (12) extends essentially in the longitudinal direction of the needle.

5. The needle according to claim 3 or 4, wherein the surface capillary (12) is provided in the area of the tip (10) and the stepped shaft section (8).

6. The needle according to any one of claims 1 to 5, wherein a plurality of surface capillaries (12) is arranged on the circumference of the needle.

7. The needle according to any one of claims 1 to 6, wherein the surface capillary (12) extends spaced from the free end (18) of the needle.

8. The needle according to any one of claims 1 to 7, wherein the surface capillary (12) has a width of 30 µm to 50 µm.

9. The needle according to any one of claims 1 to 8, which is formed integrally of a uniform material.

10. The needle according to any one of claims 1 to 8, which has a base body (14) and a coating (16) at least in the area of the surface capillary (12).

11. The needle according to claim 10, wherein the base body (14) is made of metal and the coating (16) is made of plastic.

12. The needle according to any one of claims 1 to 11, wherein the surface capillary (12) has essentially constant cross-section dimensions along its length.

13. The needle according to any one of claims 2 to 12, having a holding section (4) at an end section opposite to the tip (10) and being receivable in a holding element.

14. The needle according to claim 13, wherein the holding section (4) is formed as needle head having a greater diameter compared to the needle shaft (6).

15. A needle array with at least one needle (2) according to any one of claims 1 to 14.

16. A robot, particularly for transferring biological material, with at least one needle (2) according to any one of claims 1 to 14 or a needle array according to claim 15.

17. The robot according to claim 16, which is a picking and/or spotting robot.

18. A method for transferring biological material comprising the steps:
a) providing the biological material;
b) providing at least one needle (2) according to any one of claims 1 to 14 or at least a needle array according to claim 15, wherein the needle or needle array has at least one surface capillary (12);
c) bringing into contact the needle or the needle array with the biological material; and
.d) transferring of the biological material.

19. The method according to claim 18, wherein the needle (2) is provided at a robot.

20. The method according to claim 19, wherein the robot is a picking and/or spotting robot.

21. The method according to any one of claims 18 to 20, wherein the biological material is arranged in a pattern and is brought into contact with a plurality of needles (2) being arranged in the same pattern.

22. The method according to claim 21, wherein the pattern corresponds to the arrangement of wells of a microtiterplate or reaction vessels having a correspondingly regular arrangement.

23. The method according to any one of claims 18 to 22, wherein the biological material comprises nucleic acids, (poly)peptides or transformed host organisms.

24. The method according to claim 23, wherein the transformed host organisms are yeasts, preferably pistoria or saccharomyces cells, bacteria, especially E.coli, insect cells, preferably cells of spodoptera frugiperda, fungi cells, preferably of aspergilus, plant cells or mammal cells.

25. The method according to any one of claims 18 to 24, wherein the biological material is arranged on liquid or solid carrier.

26. The method according to claim 25, wherein the liquid carrier is a culture medium, a medium for storing biological material, a reaction buffer or a colorizing solution.

27. The method according to claim 25, wherein the solid carrier is a nitrocellulose membrane, a polyvenylidene fluoride membrane or a glass carrier.

28. A method for manufacturing a needle, particularly according to any one of claims 1 to 14, particularly for a method according to any one of claims 18 to 27, comprising the steps:
a) providing a needle-shaped body; and
b) providing at least one surface capillary (12) on said body.

29. The method according to claim 28, wherein the body is a needle with a shaft (6) and a tip (10) and the surface capillary (12) is provided in at least a part of the shaft (6).

30. The method according to claims 28 or 29, wherein the body mentioned in step a) forms a base body (14) and is provided at least in the area of the surface capillary (12) with a coating (16) in which the surface capillary (12) is formed.

31. The method according to claim 30, wherein the base body (14) is made of metal and the coating is made of plastic.

32. The method according to any one of the claims 28 to 31, wherein the surface capillary (12) is made by a laser processing.

33. The method according to any one of claims 28 to 32, wherein the surface capillary (12) is provided essentially in the longitudinal direction of the body.

34. The method according to any one of claims 28 to 33, wherein the surface capillary (12) is provided spaced from the free end (18) of the body.

## Revendications

1. Aiguille pour le transfert de liquides, en particulier de matière biologique, avec au moins un tube capillaire de surface (12).

2. Aiguille selon la revendication 1, formée d'une tige (6) et d'une pointe (10).

3. Aiguille selon la revendication 2 avec un segment de tigne èchollonné (8) sur un segment d'extrémité, tourné vers la pointe (10), de l'aiguille (2).

4. Aiguille selon l'une des revendications 1 à 3, moyennant quoi le tube capillaire de surface (12) s'étend principalement dans le sens longitudinal de l'aiguille.

5. Aiguille selon la revendication 3 ou 4, moyennant quoi les tubes capillaires de surface (12) sont formés dans la zone de la pointe (10) et du segment de tige échollonné (8).

6. Aiguille selon l'une des revendications 1 à 5, moyennant quoi plusieurs tubes capillaires de surface (12) sont disposés sur le périmètre de l'aiguille.

7. Aiguille selon l'une des revendications 1 à 6, moyennant quoi le tube capillaire de surface (12) s'étend à distance de l'extrémité libre (18) de l'aiguille.

8. Aiguille selon l'une des revendications 1 à 7, moyennant quoi le tube capillaire de surface (12) présente une largeur de 30 µm à 50 µm.

9. Aiguille selon l'une des revendications 1 à 8 qui est intégralement formée d'un matériau homogène.

10. Aiguille selon l'une des revendications 1 à 8 qui comprend un corps de base (14) et un revêtement (16) prévu au moins dans la zone du tube capillaire de surface (12).

11. Aiguille selon la revendication 10, moyennant quoi le corps de base (14) est constitué de métal et le revêtement (16) de matière plastique.

12. Aiguille selon l'une des revendications 1 à 11, moyennant quoi le tube capillaire de surface (12) comprend sur sa longueur des dimensions de section transversale essentiellement constantes.

13. Aiguille selon l'une des revendications 2 à 12 avec une zone de support (4), pour le logement dans un élément de support, prévue sur le segment d'extrémité opposé à la pointe (10).

14. Aiguille selon la revendication 13, moyennant quoi la zone de support (4) en tant que tête de l'aiguille présente un diamètre supérieur à celui de la tige de l'aiguille (6).

15. Matrice d'aiguille avec au moins une aiguille (2) selon l'une des revendications 1 à 14.

16. Robot, en particulier pour le transfert de matière biologique, avec au moins une aiguille (2) selon l'une des revendications 1 à 14 ou une matrice d'aiguille selon la revendication 15.

17. Robot selon la revendication 16 configuré comme un robot de prélèvement et/ou de retouche.

18. Procédé de transfert de matière biologique comprenant les étapes suivantes :
a) Fourniture de matière biologique ;
b) Fourniture d'au moins une aiguille (2) selon l'une des revendications 1 à 14, ou d'au moins une matrice d'aiguille selon la revendication 15 comprenant au moins un tube capillaire de surface (12) ;
c) Mise en contact de l'aiguille ou de la matrice d'aiguille avec la matière biologique ; et
d) Transfert de la matière biologique.

19. Procédé selon la revendication 18, dans lequel l'aiguille (2) est prévue sur un robot.

20. Procédé selon la revendication 19, dans lequel le robot est un robot de prélèvement et/ou de retouche.

21. Procédé selon l'une des revendications 18 à 20, dans lequel la matière biologique est disposée dans un modèle et mise en contact avec plusieurs aiguilles (2) placées selon le même modèle.

22. Procédé selon la revendication 21, dans lequel le modèle correspond à la disposition des cavités d'un plateau microtitre ou au modèle de réacteurs conçus dans une disposition régulière correspondante.

23. Procédé selon l'une des revendications 18 à 22, dans lequel la matière biologique comprend de l'acide nucléique, des (poly)peptides ou des organismes-hôtes transformés.

24. Procédé selon la revendication 23, dans lequel les organismes-hôtes transformés sont des levures, de préférence des levures Pistoria ou des cellules de saccharomyces, des bactéries, de préférence des bactéries E. coli, des cellules d'insectes, de préférence des cellules de Spodoptera frugiperda, des cellules de champignons, de préférence des cellules d'aspergilus, des cellules de plantes ou des cellules de mammifères.

25. Procédé selon l'une des revendications 18 à 24, dans lequel la matière biologique est placée sur un support liquide ou solide.

26. Procédé selon la revendication 25, dans lequel le support liquide est un bouillon de cultures, un milieu pour le stockage de la matière biologique, un tampon de réaction ou une solution colorante.

27. Procédé selon la revendication 25, dans lequel le support solide est une membrane en nitrocellulose, une membrane en polyfluorure de vinylidène ou un support en verre.

28. Procédé de fabrication d'une aiguille, en particulier selon l'une des revendications 1 à 14, en particulier pour un procédé selon l'une des revendications 18 à 27 comprenant les étapes suivantes :
a) Fourniture d'un corps en forme d'aiguille ; et
b) Prévision d'au moins un tube capillaire de surface (12) sur le corps.

29. Procédé selon la revendication 28, dans lequel le corps est une aiguille avec une tige (6) et une pointe (10) et le tube capillaire de surface (12) est prévu au moins dans la zone de la tige (6).

30. Procédé selon la revendication 28 ou 29, dans lequel le corps mentionné dans l' étape a) forme un corps de base (14) et est doté d'un revêtement (16) au moins dans la zone du tube capillaire de surface (12) dans lequel est formé le tube capillaire de surface (12).

31. Procédé selon la revendication 30, dans lequel le corps de base (14) est en métal et le revêtement (16) est en matière plastique.

32. Procédé selon l'une des revendications 28 à 31, dans lequel le tube capillaire de surface (12) est fabriqué par traitement laser.

33. Procédé selon l'une des revendications 28 à 32, dans lequel le tube capillaire de surface (12) est prévu essentiellement dans le sens longitudinal du corps.

34. Procédé selon l'une des revendications 28 à 33, dans lequel le tube capillaire de surface (12) est prévu à distance de l'extrémité libre (18) du corps.
